(19) Europäisches Patentamt
European Patent Office
Office européen des brevets

(11) Veröffentlichungsnummer: **0 396 078 A2**

# (12) EUROPÄISCHE PATENTANMELDUNG

(21) Anmeldenummer: **90108212.3**

(22) Anmeldetag: **30.04.90**

(51) Int. Cl.⁵: **C07D 405/12, A01N 43/653, A01N 43/56**

(30) Priorität: **03.05.89 DE 3914632**

(43) Veröffentlichungstag der Anmeldung: **07.11.90 Patentblatt 90/45**

(84) Benannte Vertragsstaaten: **AT BE CH DE DK ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Baus, Ulf, Dr.**
**Keltenweg 10**
**D-6915 Dossenheim(DE)**
Erfinder: **Reuther, Wolfgang, Dr.**
**Am Pferchelhang 16**
**D-6900 Heidelberg(DE)**
Erfinder: **Lorenz, Gisela, Dr.**
**Erlenweg 13**
**D-6730 Neustadt(DE)**
Erfinder: **Ammermann, Eberhard, Dr.**
**Sachsenstrasse 3**
**D-6700 Ludwigshafen(DE)**

## (54) 1-Hydroxi-azolverbindungen und diese enthaltende Fungizide.

(57) Verbindungen der allgemeinen Formel I

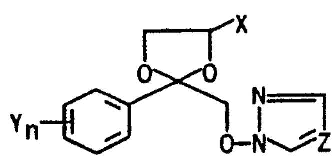

in der
X Alkyl, Aryl, Aryloxi, Alkyloxi,
Y Wasserstoff, Alkyl, Alkyloxi, Halogen, Aryl, Aryloxi,
n 0 bis 5 und
Z CH oder N bedeuten,
und diese Verbindungen enthaltende Fungizide.

EP 0 396 078 A2

## 1-Hydroxi-azolverbindungen und diese enthaltende Fungizide

Die vorliegende Erfindung betrifft neue N-Hydroxi-azolderivate, ihre Salze und Metallkomplexe, ein Verfahren zu ihrer Herstellung und ihre Verwendung als Fungizide.

Es wurde nun gefunden, daß Verbindungen der allgemeinen Formel I

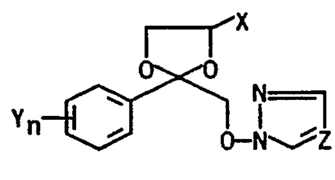

(I),

in der

X Alkyl mit 1 bis 9 C-Atomen, einen Alkoxi- oder Aryloxirest oder einen Arylrest, der gegebenenfalls durch Halogen, Phenyl, Aryloxi oder Alkyloxi substituiert sein kann,

Y Wasserstoff, Alkyl, Alkyloxi, Halogen, Aryl, Aryloxi,

n 0 bis 5 und

Z CH oder N bedeuten,

und ihre pflanzenverträglichen Säureadditionssalze und Metallkomplexe eine überraschend gute fungizide Wirkung haben.

X bedeutet beispielsweise $C_1$-$C_9$-Alkyl, insbesondere $C_1$-$C_4$-Alkyl (Methyl, Ethyl, tert.-Butyl, Pentyl, Hexyl, Heptyl, Octyl, Nonyl), $C_1$-$C_4$-Alkoxi (Methoxi, Ethoxi, tert.-Butoxi), Halogen (Cl, Br, F), Aryloxi (Phenoxi) oder Aryl (Phenyl), das gegebenenfalls 1- bis 5-fach (1- bis 3-fach) durch die gleichen oder verschiedenen Reste Halogen (Fluor, Chlor, Brom), Phenyl, Aryloxi (Phenoxi), Alkyloxi mit 1 bis 4 C-Atomen (Methoxy, Ethoxy, Propoxy) substituiert sein kann, z.B. 4-Chlorphenyl, 2,4-Dichlorphenyl, 3-Methoxyphenyl, 4-Chlor-2-methylphenyl, 2,6-Dimethylphenyl.

Y bedeutet beispielsweise $C_1$-$C_4$-Alkyl (Methyl, Ethyl, Propyl), $C_1$-$C_4$-Alkoxi (Methoxi, Ethoxi), Halogen (Fluor, Chlor, Brom), Aryl (Phenyl), Aryloxi (Phenoxi). n bedeutet 0, 1, 2, 3, 4 oder 5, wobei für n größer als 1 die Reste Y gleich oder verschieden sind, z.B. 2,4-Dichlor- oder 4-Chlor-2-methyl.

Salze sind beispielsweise die pflanzenverträglichen Säureadditionssalze, z.B. Salze mit anorganischen oder organischen Säuren, wie beispielsweise die Salze der Salzsäure, Bromwasserstoffsäure, Salpetersäure, Oxalsäure, Essigsäure, Schwefelsäure, Phosphorsäure oder Dodecylbenzolsulfonsäure. Die Wirksamkeit der Salze geht auf das Kation zurück, so daß die Wahl des Anions im allgemeinen beliebig ist.

Ferner lassen sich die Verbindungen der Formel I nach bekannten Methoden in Metallkomplexe überführen. Das kann durch Umsetzung dieser Verbindungen mit Metallsalzen, z.B. Salzen der Metalle Kupfer, Zink, Eisen, Mangan oder Nickel, beispielsweise Kupfer(II)-chlorid, Zink(II)-chlorid, Eisen(III)-chlorid, Kupfer(II)-nitrat, Mangan(II)-chlorid oder Nickel(II)-bromid erfolgen.

Es wurde ferner gefunden, daß sich die Verbindungen der allgemeinen Formel I sehr leicht und in guten Ausbeuten durch Reaktion von 1-Hydroxi-azolen mit den Verbindungen der allgemeinen Formel II

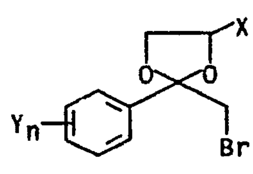

(II),

in der X, Y und n die in der allgemeinen Formel I angegebenen Bedeutungen haben, herstellen lassen. Die Verbindungen der allgemeinen Formel II sind bekannt oder lassen sich durch bekannte Verfahren herstellen (z.B. DE-OS 2 551 560).

1-Hydroxi-1,2,4-triazol läßt sich wie folgt herstellen:

103,5 g (1,5 mol) 1-H-1,2,4-Triazol wurden in 1344 g (12 mol) 50 %igem wäßrigem Kaliumhydroxid gelöst. Unter Eiskühlung wurden 340 g (3 mol) 30 %iges $H_2O_2$ und portionsweise 555 g (3,75 mol) Phthalsäureanhydrid zugegeben und 2 Stunden bei Raumtemperatur (20 bis 30 ° C) gerührt. Anschließend wurde mit ca. 35 %iger Schwefelsäure auf einen pH-Wert unter 1,5 angesäuert, der entstandene Niederschlag abgesaugt und das Filtrat wie üblich aufgearbeitet. Man erhielt 19 g 1-Hydroxi-1,2,4-triazol Fp.: 132 ° C. Das

ist eine Ausbeute von 15 % der Theorie.

Die Umsetzungen erfolgen z.B. in einem inerten organischen Lösemittel wie Tetrahydrofuran, Dimethylsulfoxid, Diethylether, vorzugsweise Dimethylformamid in Gegenwart einer Base wie Triethylamin, Tributylamin, NaOH, Natriumcarbonat oder Pyridin bei Temperaturen zwischen 0 und 200 °C, vorzugsweise bei 150 °C. Vorteilhafter ist es, die 1-Hydroxiazole vor der Zugabe der Bromverbindung in an sich bekannter Weise in ein Alkaliderivat zu überführen, z.B. durch Verwendung von Basen wie Butyllithium oder NaOH/Molekularsieb oder Na-methylat. Als vorteilhaft hat sich bei der Umsetzung die Zugabe einer katalytischen Menge Alkaliiodid erwiesen.

Herstellungsbeispiel

3,4 g (40 mmol) 1-Hydroxi-1,2,4-triazol wurden in Dimethylformamid mit 2,55 g KOH (88 %ig) versetzt. Das entstehende Reaktionswasser wurde mit Ethanol im Rotationsverdampfer entfernt. Es wurden 60 ml DMF und eine Spatelspitze NaI zugegeben. Unter Rühren wurden danach 12,8 g (40 mmol) 2-Brom-1-(2,4-dichlorphenyl)-1-(4-ethyl-1,3-dioxolan-2-yl)-ethan zugegeben und die Lösung 5 h auf 160 °C erwärmt. Nach beendeter Reaktion wurde das DMF im Rotationsverdampfer entfernt und der Rückstand in Toluol aufgenommen und mit Wasser gewaschen. Nach Trocknen der Toluolphase und Entfernen des Lösemittels erhielt man 9,7 g (70 %) eines viskosen Öls, das durch Säulenchromatographie gereinigt wurde (Verbindung Nr. 1).

| | | | | |
|---|---|---|---|---|
| Analyse: $C_{14}H_{15}Cl_2N_3O_3$ ber.: | C 48,8 | H 4,4 | Cl 20,6 | N 12,2 |
| (344.19) gef.: | C 49.2 | H 4,7 | Cl 20,1 | N 12,6 |

In entsprechender Weise wurden erhalten:

| Nr. | X | Y | Z | n | phys. Daten |
|---|---|---|---|---|---|
| 1 | ethyl | 2,4-DiCl | N | 2 | Öl |
| 2 | propyl | 2,4-DiCl | N | 2 | Öl |
| 3 | methyl | 2,4-DiCl | N | 2 | Öl |
| 4 | propyl | 2,4-DiCl | CH | 2 | Öl |
| 5 | ethyl | 2,4-DiCl | CH | 2 | Öl |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,

Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor Pilzbefall zu schützenden Pflanzen, Saatgüter, Materialien oder der Erdboden mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungs zwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Bei der Saatgutbehandlung werden im allgemeinen Wirkstoffmengen von 0,001 bis 50 g, vorzugsweise 0,01 bis 10 g, je Kilogramm Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 1 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 2 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 1 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 2 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 1 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 1 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 2 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiel

Wirksamkeit gegen Pyricularia oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 bis 24 °C und 95 bis 99 % relativer Luftfeuchtigkeit aufgestellt. Nach 6 Tagen wurde das Ausmaß des Krankheitsbefalls ermittelt.

Das Ergebnis zeigt, daß der Wirkstoff 2 bei der Anwendung als 0,05 %ige (Gew.%) Spritzbrühe eine gute fungizide Wirkung zeigt (90 %).

## Ansprüche

1. Verbindungen der allgemeinen Formel I

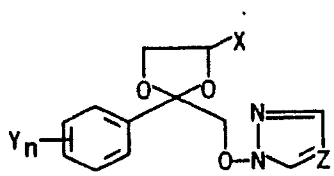

(I),

in der

X Alkyl mit 1 bis 9 C-Atomen, einen Alkoxi- oder Aryloxirest oder einen Arylrest, der gegebenenfalls durch Halogen, Phenyl, Aryloxi oder Alkyloxi substituiert sein kann,

Y Wasserstoff, Alkyl, Alkyloxi, Halogen, Aryl, Aryloxi,

n 0 bis 5 und

Z CH oder N bedeuten,

und ihre pflanzenverträglichen Säureadditionssalze und Metallkomplexe.

2. Verfahren zur Herstellung von Verbindungen der allgemeinen Formel I gemäß Anspruch 1, dadurch gekennzeichnet, daß man Verbindungen der allgemeinen Formel II

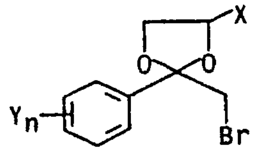

(II),

in der X, Y und n die in Anspruch 1 angegebenen Bedeutungen haben, mit 1-Hydroxi-1,2,4-triazol umsetzt.

3. Fungizide Mittel, gekennzeichnet durch einen Gehalt an einem Trägerstoff und einer fungizid wirksamen Menge einer Verbindung der Formel

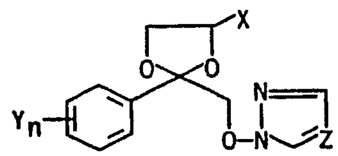

in der

X Alkyl mit 1 bis 9 C-Atomen, einen Alkoxi- oder Aryloxirest oder einen Arylrest, der gegebenenfalls durch Halogen, Phenyl, Aryloxi oder Alkyloxi substituiert sein kann,

Y Wasserstoff, Alkyl, Alkyloxi, Halogen, Aryl, Aryloxi,

n 0 bis 5 und

Z CH oder N bedeuten,

oder deren pflanzenverträglichem Säureadditionssalz oder Metallkomplex.

4. Verfahren zur Bekämpfung von Pilzen, dadurch gekennzeichnet, daß man eine fungizid wirksame Menge einer Verbindung der Formel

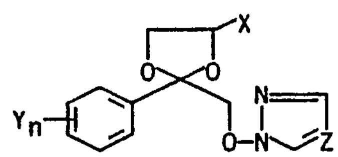

in der

X Alkyl mit 1 bis 9 C-Atomen, einen Alkoxi- oder Aryloxirest oder einen Arylrest, der gegebenenfalls durch Halogen, Phenyl, Aryloxi oder Alkyloxi substituiert sein kann,

Y Wasserstoff, Alkyl, Alkyloxi, Halogen, Aryl, Aryloxi,

n 0 bis 5 und

Z CH oder N bedeuten,

oder deren pflanzenverträglichem Säureadditionssalz oder Metallkomplex auf die Pilze oder die vom Pilzbefall bedrohten Pflanzen, Saatgüter, Materialien oder den Erdboden einwirken läßt.

5. Verbindung der Formel I gemäß Anspruch 1, dadurch gekennzeichnet, daß X Ethyl, Y 2,4-Dichlor, Z N und n 2 bedeutet.

6. Verbindung der Formel I gemäß Anspruch 1, dadurch gekennzeichnet, daß X Propyl, Y 2,4-Dichlor, Z N und n 2 bedeutet.